# EUROPEAN PATENT SPECIFICATION

(11) **EP 4 582 128 B1**
(45) Date of publication and mention of the grant of the patent: **25.11.2026**
(21) Application number: 23877366.7
(22) Date of filing: 13.10.2023
(51) Int. Cl.: A61M 25/04, A61F 2/915

(54) **DISTAL STABILIZER**
DISTALER STABILISATOR
STABILISATEUR DISTAL

(30) Priority: 14.10.2022 JP 2022165641
(43) Date of publication of application: 09.07.2025
(73) Proprietor: Bolt Medical Inc., Tokyo 101-0021 (JP)
(72) Inventor: INUZUKA Naoki, Tokyo 103-0023 (JP)
(74) Representative: Appleyard Lees IP LLP
(86) International application number: PCT/JP2023/037233
(87) International publication number: WO 2024/080365

(56) References cited:
- EP-A1- 4 014 933
- WO-A1-2022/004850
- WO-A1-2022/004850
- WO-A1-2022/034905
- JP-A- 2015 181 746

## Description

### TECHNICAL FIELD

The present invention relates to a distal stabilizer for use for catheter delivery in a biological lumen.

### BACKGROUND ART

Certain treatments are administered by means of a catheter guided into a biological lumen, such as a patient's artery, so that a distal end of the catheter is placed in a vicinity of a target position. In this connection, the inner path of the catheter is used to deliver a treatment device to the target position, or the catheter itself is used as a treatment device. For example, Patent Document 1 discloses a distal stabilizer (anchoring device) including a delivery wire and a locking stent for anchoring that is joined to the distal end of the delivery wire. Upon being released from a microcatheter, the locking stent expands to be anchored to the inner wall of a biological lumen, so that a treatment catheter externally fitted over the microcatheter can be delivered to the vicinity of a target position.

WO 2022/004850 A1 discloses a distal stabilizer that can be engaged with the inner wall of a biological lumen, and can be easily resheathed to a catheter.

### Citation List

### Patent Document

Patent Document 1: US Patent No. 9682216

### DISCLOSURE OF THE INVENTION

### Problems to be Solved by the Invention

Intracranial blood vessels such as an anterior cerebral artery (ACA), an anterior communicating artery (Acom), a middle cerebral artery (MCA), etc. are known as parts where an aneurysm is likely to form. Since these blood vessels have a small diameter and bent shape, it is difficult to deliver a large-diameter catheter therein, and only a small-diameter catheter having a small inner diameter of about 0.42 mm (about0.0165 inches ) can be usually delivered to the vicinity of an aneurysm forming in such a blood vessel. On the other hand, an aneurysm stent and a flow diverter are used as aneurysm treatment devices. In order to deliver these treatment devices to the vicinity of a target position, it is necessary to use a large-diameter catheter having an inner diameter of about 0.53 mm (about0.021 inches ) to about 0.69 mm (about 0.027 inches).

In a case where the above treatment devices are to be delivered to an aneurysm in a narrow blood vessel, use of a locking stent (distal stabilizer) as an anchoring device is necessitated because it is difficult or impossible to deliver a large-diameter catheter by means of a guidewire, which is a common approach. To this end, a small-diameter catheter is initially delivered to on a distal side of the aneurysm, and then, the locking stent is anchored to on the distal side of the aneurysm. This procedure requires insertion of the locking stent (distal stabilizer) into the small-diameter catheter. On the other hand, in a case of increasing the expansion force of the locking stent in order for the locking stent to have a frictional force with a blood vessel wall that is necessary for delivery of a large-diameter catheter, there is concern that the blood vessel wall may be damaged and the slidability of the locking stent with respect to the inner wall of the catheter tends to deteriorate. An increase in the frictional force with the blood vessel wall requires an increase in the mesh density of the locking stent. However, when a locking stent having a high mesh density is inserted into a small-diameter catheter, the wires (struts) forming the locking stent overlap with each other, resulting in an increase in the diameter of the locking stent, whereby the slidability with respect to the inner wall of the catheter tends to deteriorate. These phenomena are particularly significant, for example, for a locking stent having an open cell structure that is highly protective of the blood vessel wall.

An object of the present invention is to provide a distal stabilizer with an open cell structure, which has a frictional force with a blood vessel wall necessary for the distal stabilizer as an anchoring device for delivering a large-diameter catheter, and exhibits an excellent slidability when reduced in diameter and inserted into a small-diameter catheter.

### Means for Solving the Problems

The present invention relates to a distal stabilizer for use for catheter delivery in a biological lumen according to claim 1. Optional features are set out in the dependent claims. In examples, the distal stabilizer may include: a linear delivery member; and a cylindrical part extending from a distal end of the linear delivery member and lockable to an inner wall of the biological lumen. The cylindrical part has a structure in which cells each having a shape surrounded by wire-shaped members are arranged along a longitudinal direction, and at least one of the cells is an open cell having at least one protruding free end. A wire-shaped member, which extends in an arrangement direction of the cells and is one of wire-shaped members that form the at least one protruding free end, is disposed substantially parallel to one of wire-shaped members forming another cell that is adjacent side by side to the open cell with respect to the arrangement direction.

In the distal stabilizer, the wire-shaped member forming the protruding free end of the cell and extending in the arrangement direction, when the wire-shaped member is in a natural state, may intersect at an angle of 0° to 15° with one of the wire-shaped members forming another cell adjacent in the lateral direction with respect to the arrangement direction.

In the distal stabilizer, the cylindrical part may have a structure in which cells each having a shape surrounded by wire-shaped members are arranged helically with respect to the longitudinal direction.

In the distal stabilizer, the cylindrical part may be used by being inserted into a catheter having an inner diameter of 0.43 mm (0.017 inches) or less.

### Effects of the Invention

The present invention can provide a distal stabilizer having an open cell structure, which has a frictional force with a blood vessel wall necessary for the distal stabilizer as an anchoring device for delivering a large-diameter catheter and exhibits an excellent slidability when reduced in diameter and inserted into a small-diameter catheter.

### BRIEF DESCRIPTION OF THE DRAWINGS

FIG. 1 is a diagram illustrating an overall configuration of a delivery system 10 including a distal stabilizer 1 according to an embodiment;
FIG. 2 is a developed view illustrating a state in which a locking stent 2 is developed virtually onto a plane;
FIG. 3A is an enlarged view of a region A in FIG. 2;
FIG. 3B is an enlarged view illustrating another embodiment of the region A in FIG. 2;
FIG. 4 is a schematic diagram illustrating an example of a surgical procedure performed using the delivery system 10 including the distal stabilizer 1;
FIG. 5 is a schematic diagram illustrating an example of a surgical procedure performed using the delivery system 10 including the distal stabilizer 1;
FIG. 6 is a schematic diagram illustrating an example of a surgical procedure performed using the delivery system 10 including the distal stabilizer 1;
FIG. 7 is a schematic diagram illustrating an example of a surgical procedure performed using the delivery system 10 including the distal stabilizer 1;
FIG. 8 is a schematic diagram illustrating an example of a surgical procedure performed using the delivery system 10 including the distal stabilizer 1;
FIG. 9 is a schematic diagram illustrating an example of a surgical procedure performed using the delivery system 10 including the distal stabilizer 1;
FIG. 10 is a schematic diagram illustrating an example of a surgical procedure performed using the delivery system 10 including the distal stabilizer 1; and
FIG. 11 is a schematic diagram illustrating an example of a surgical procedure performed using the delivery system 10 including the distal stabilizer 1.

### PREFERRED MODE FOR CARRYING OUT THE DISCLOSURE

Embodiments of a distal stabilizer according to the present invention will be described below. It should be noted that all of the drawings attached to the present disclosure are schematic diagrams, and the shape, the scale, the vertical and horizontal dimensional ratio, and the like of each part are changed or exaggerated from actual ones in consideration of ease of understanding and the like. For example, illustrated catheters and other components appear to have a shorter dimension in a longitudinal direction and a longer (larger) dimension in a radial direction. In the present specification and the accompanying documents, terms specifying shapes, geometric conditions, and levels thereof, examples of which include "direction", "orthogonal/perpendicular", etc., each encompass not only a strict meaning of the respective terms, but also an extent which can be regarded as approximately being in the direction and an extent which can be regarded as nearly orthogonal/perpendicular, etc. In the present specification, the longitudinal direction in a state in which a distal stabilizer 1 is extended linearly may also be referred to as an "axial direction LD" or simply as an "axial direction". In the longitudinal direction LD, a proximal side close to a practitioner is denoted by "D1", and a distal side away from the practitioner is denoted by "D2".

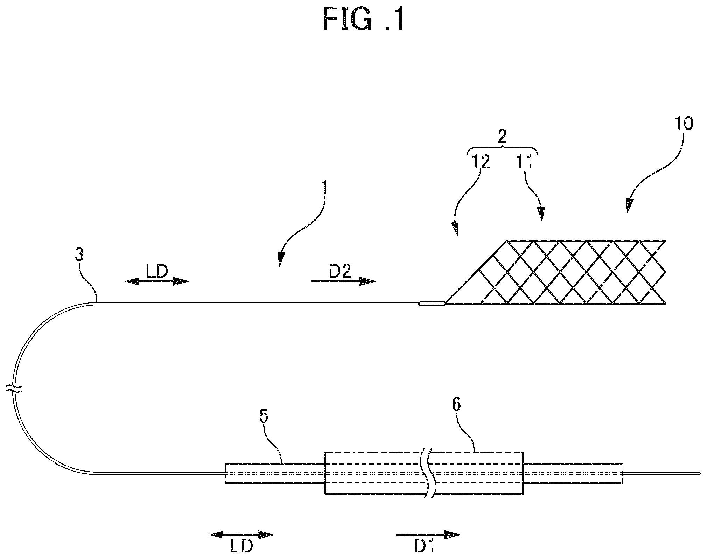

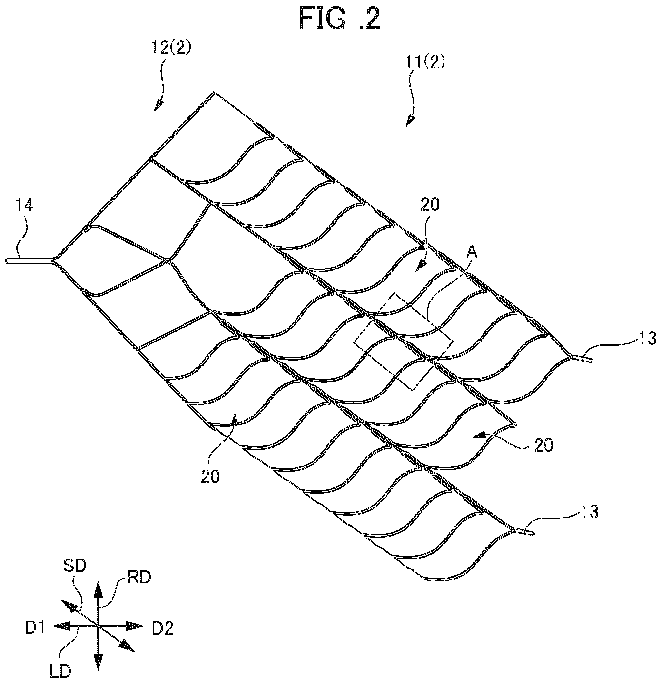

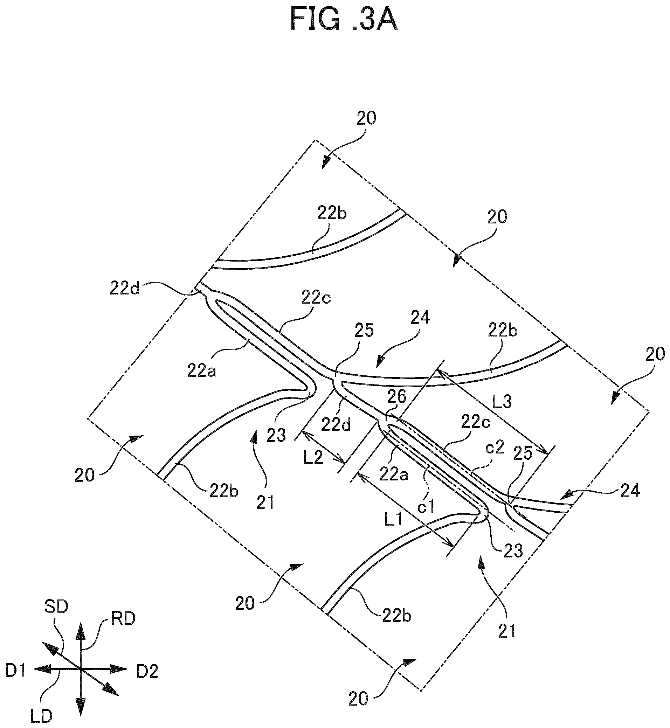

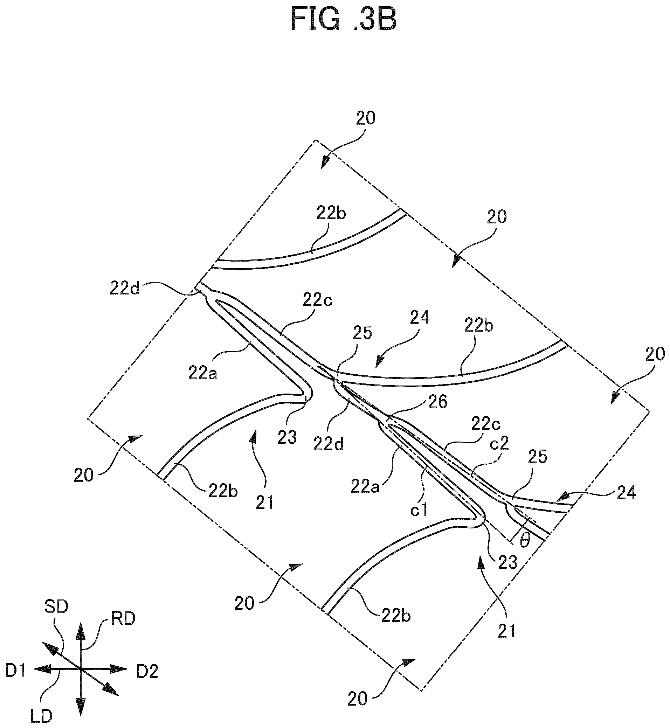

FIG. 1 is a diagram illustrating an overall configuration of a delivery system 10 including a distal stabilizer 1 according to the present embodiment. FIG. 2 is a developed view illustrating a state in which a locking stent 2 is developed virtually onto a plane. FIG. 3A is an enlarged view of a region A in FIG. 2. FIG. 3B is an enlarged view illustrating another embodiment of the region A in FIG. 2. Each of FIGS. 2, 3A, and 3B is a developed view illustrating the locking stent 2 in a natural state (described later).

The delivery system 10 illustrated in FIG. 1 can be used to deliver a treatment device in a blood vessel that is a biological lumen, such as an intracranial blood vessel, examples of which include an anterior cerebral artery (ACA), an anterior communicating artery (Acom), and a middle cerebral artery (MCA), and the like. Furthermore, the blood vessel of the biological lumen in which the delivery system 10 is used is not limited to the above examples. The blood vessel as the biological lumen may include, for example, a coronary artery or vein, a blood vessel (artery or vein) of an upper or lower limb, an organ, etc., in addition to the intracranial blood vessels. In the following description, the biological lumen is also referred to as a "blood vessel".

As illustrated in FIG. 1, the delivery system 10 includes the distal stabilizer 1 and a plurality of catheters including a first catheter 5 and a second catheter 6. The first catheter 5 is, for example, a catheter called a microcatheter. The second catheter 6 is externally fitted over the first catheter 5. The second catheter 6 is a large-diameter catheter. The diameter of the first catheter 5 is set according to: a target position at which the second catheter 6 is to be delivered; and the inner diameter and the degree of bend of a biological lumen constituting a path leading to the target position. It is not particularly limited, but the inner diameter of the first catheter 5 may be preferably 0.4318 mm (0.017 inches) or less, and more preferably 0.42mm (0.0165 inches) or less. One or more other catheters (not shown) that are externally fitted over the second catheter 6 may be used as necessary. In general, using multiple catheters may make it possible to ultimately insert and advance a catheter having a large inner diameter into a biological lumen.

The distal stabilizer 1 is a device used for catheter delivery in a biological lumen. The distal stabilizer 1 includes a locking stent (cylindrical part) 2 and a delivery wire (linear delivery member) 3. The locking stent 2 is an anchoring device that is inserted into the first catheter 5 while being in a reduced diameter state, and expands to be locked to the inner wall of the body lumen upon being released from the first catheter 5 in a blood vessel. The locking stent 2 is coupled to, and extends from, the distal end of the delivery wire 3. As illustrated in FIG. 1, the locking stent 2 includes a main body 11 and an antenna portion 12. The main body 11 is formed in a cylindrical shape and has a mesh pattern structure, which will be described later. FIG. 1 depicts the configuration of the main body 11 in a simplified manner. The antenna portion 12 is where a proximal side D1 portion of the main body 11 converges to the delivery wire 3.

As illustrated in FIG. 2, a distal marker 13 is provided at a distal side D2 end of the locking stent 2. A proximal marker 14 is provided at a proximal side D1 end of the locking stent 2. Each marker is made of an X-ray opaque material. The distal marker 13 serves as a mark to check the position of the distal side D2 end of the locking stent 2. The proximal marker 14 serves as a mark to check the position of the proximal side D1 end of the locking stent 2.

The main body 11 has a structure in which a plurality of cells 20 are arranged along the longitudinal direction LD of the locking stent 2. In the present embodiment, the main body 11 has a mesh pattern structure in which the plurality of cells 20 each including an open cell portion 21 and a closed cell portion 24, which will be described later, are arranged helically with respect to the longitudinal direction LD of the locking stent 2. As illustrated in FIG. 2, the plurality of cells 20 are arranged helically with respect to a cell arrangement direction (cell arrangement direction as designed) SD that is inclined with respect to the longitudinal direction LD. In the present embodiment, as illustrated in FIG. 2, the cell arrangement direction SD is inclined upward in a leftward direction from the distal side D2 toward the proximal side D1 of the locking stent 2, but this is a non-limiting example. The cell arrangement direction SD may be inclined upward in a rightward direction (inverted direction with respect to FIG. 2) from the distal side D2 toward the proximal side D1 of the locking stent 2. The term "along/in the cell arrangement direction SD" refers to being parallel or substantially parallel to the cell arrangement direction SD. The term "substantially parallel" refers to, for example, a range in which an angle between an arrangement direction of the cells 20 and the cell arrangement direction SD is about 1° to 15°. In the locking stent 2, it may be desirable that an angle at which the longitudinal direction LD intersects with the cell arrangement direction SD is less than 45°.

The main body 11 is designed to have a surface area that allows the main body 11 as an anchoring device to have a frictional force with a blood vessel wall necessary for delivery of a large-diameter catheter (e.g., the second catheter 6). Specifically, a surface area S of the mesh pattern (area excluding openings of the cells) of the main body 11 in an expanded diameter state is set to 5% or more and 20% or less of a surface area S₀ of a virtual cylinder having the same dimensions in the longitudinal direction and the radial direction as the main body 11. Setting the surface area S of the main body 11 to 5% or more with respect to the surface area S₀ of the virtual cylinder makes it possible to obtain the frictional force with the blood vessel wall necessary for delivery of the large-diameter catheter. Setting the surface area S of the main body 11 to 20% or less with respect to the surface area S₀ of the virtual cylinder makes it possible to reduce the overlap of the struts with each other when the locking stent 2 is reduced in diameter. The main body 11 is designed to have: an expansion force of 0.015 N/mm or greater and 0.06 N/mm or less per unit length in a reduced diameter state in which an outer diameter is 1.5 mm; and an expansion force of 0.1 N/mm or greater and 0.3 N/mm or less per unit length in a reduced diameter state in which an outer diameter is 0.42 mm. Setting the expansion force per unit length to 0.06 N/mm or less in the reduced diameter state with the outer diameter of 1.5 mm makes it possible to reduce the risk of the locking stent 2 straightening and damaging a bent blood vessel. Setting the expansion force per unit length to 0.3 N/mm or less in the reduced diameter state with the outer diameter of 0.42 mm reduces the sliding resistance, thereby allowing for suitable use in a microcatheter of 0.42mm (0.0165 inches) or less.

The locking stent 2 of the present embodiment undergoes measurement of a tensile load of 1.0 N or greater and 4.0 N or less under the following measurement conditions.
Equipment used:
   microcatheter: SL10 (manufactured by Excelsior Stryker);
   digital force gauge (push-pull gauge);
   retraction device;
   thermostatic chamber; and
   thermometer.
Test conditions:
   speed: 100 mm/min;
   pull distance: effective length plus 10 mm; and
   test temperature: 37±2°C.
Test method:
   checking whether the temperature of the thermostatic chamber is 37±2°C with the thermometer;
   placing the microcatheter such that the distal end is at the position of a cerebral blood vessel in a model anatomically imitating blood vessels of a human body and maintained at 37±2°C;
   inserting the locking stent from the proximal side of the microcatheter until the locking stent is entirely accommodated in the microcatheter, and placing the locking stent in the cerebral blood vessel;
   connecting the digital force gauge set on the retraction device to the proximal side of the locking stent;
   fixing the microcatheter in a state in which the proximal side of the microcatheter is straightened, and pulling the locking stent toward the proximal side by the retraction device at the prescribed constant speed; and
   recording the maximum value of the tensile load measured by the digital force gauge at the time when the locking stent is pulled by a distance equivalent to the sum of the effective length plus 10 mm.
By way of the test performed under the above-described measurement conditions, it is possible to verify whether a locking stent of interest meets the slidability requirements for the locking stent of the distal stabilizer according to the present invention. In this connection, the "effective length" of the test conditions refers to a length between the distal end of one cell 20 closest to the distal side D2 and the proximal end of another cell 20 closest to the proximal side D1 in the mesh pattern structure of the locking stent.

The cell 20 is also referred to as an opening or a compartment, and is a portion surrounded by wire-shaped struts (wire-shaped members) 22 that form the mesh pattern of the main body 11. The open cell portion 21 is a portion of a cell 20 and has a protruding free end 23. In the locking stent 2 of the present embodiment, all the cells 20 are open cells each having the open cell portion 21. As illustrated in FIG. 3A, the protruding free end 23 is an end portion in which two struts 22a and 22b are connected to each other at their distal side D2 ends, and which is not connected to any other strut. The protruding free end 23 has, for example, a substantially V-shape, a substantially U-shape, or a substantially Ω-shape. It is sufficient for the protruding free end 23 to protrude not only substantially toward the distal side D2 but also in the cell arrangement direction SD, when viewed in the direction in which the locking stent 2 is inserted into the catheter. Since protruding free ends 23 are less likely to be restrained by other struts, displacement and deformation in a radial direction (direction orthogonal to the longitudinal direction LD) are less likely to be restricted. It should be noted that a protruding direction of each protruding free end 23 is not limited to the example of the present embodiment.

As illustrated in FIG. 3A, a protruding free end 23 of each cell 20 is formed by struts 22a and 22b. The strut 22a extends in the cell arrangement direction SD and is disposed substantially parallel to a strut 22c of another cell 20 adjacent side by side with respect to the cell arrangement direction SD. Here, extending in the cell arrangement direction SD means extending substantially straight in the cell arrangement direction SD. The strut 22a extending in the cell arrangement direction SD has a length L1 of 0.2 mm to 1.4 mm, for example. The length of a strut refers to a length of a portion in the extending direction of the strut, excluding portions connected to other struts. The portions connected to the other struts include, for example, a protruding free end 23, a protruding closed end 25, a vertex 26 (described later), and the like. In a case where a strut is not straight or includes a non-straight portion, the length refers to a virtual length in a state in which the strut is virtually straightened. The term "adjacent side by side with respect to the cell arrangement direction SD" refers to adjacency in a direction orthogonal or substantially orthogonal to the cell arrangement direction SD. The term "substantially parallel" means that components of interest are disposed at 0° (parallel) or at an angle of 15° or less, as will be described later. As illustrated in FIG. 3A, proximal side D1 ends of the struts 22a and 22c are connected to each other at the vertex 26. Furthermore, the proximal side D1 ends of the struts 22a and 22c are connected to a distal side D2 end of another strut 22d at the vertex 26. The strut 22d has a length L2 of, for example, 0.1 mm to 0.7 mm in the cell arrangement direction SD. The length L1 of the strut 22a and the length L2 of the strut 22d may be the same in all the cells or may be different from one cell to another. For example, the ratio of the length L1 of the strut 22a to the length L2 of the strut 22d may be preferably about 2:1, but is not limited to this ratio. For example, the ratio of the length L1 of the strut 22a to a length L3 of the strut 22c may be preferably about 1:1.2, but is not limited to this ratio, either.

In a natural state, a strut 22a, which is one of struts forming a protruding free end 23 of one cell 20 and extends in the cell arrangement direction SD, is disposed at an angle of 0° with respect to a strut 22c of another cell 20 adjacent aide by side with respect to the cell arrangement direction SD, or is disposed so as to intersect with the strut 22c at an angle of 15° or less. The natural state refers to a state in which the locking stent 2 is not reduced in diameter (no-load state). In the configuration illustrated in FIG. 3A, a strut 22a of one cell 20 and a strut 22c of another adjacent cell 20 are disposed so that a center line c1 of the strut 22a is at an angle of 0° (parallel) with respect to a center line c2 of the strut 22c. In the embodiment illustrated in FIG. 3B, the center line c1 of the strut 22aintersects with the center line c2 of the strut 22c of the adjacent cell 20 at an angle of 15° or less, for example, at an angle of 1° to 15°.

By disposing the strut 22a, which extends in the cell arrangement direction SD, substantially parallel to the strut 22c of the other adjacent cell 20, surplus spaces between the struts are reduced, thereby making it possible to arrange the struts close to each other. Consequently, when the diameter of the stent is reduced, struts 22b which have surplus spaces with respect to struts 22a are first folded, and the reduction of the diameter of the locking stent progresses while the orientation of struts 22a and 22c with respect to the cell arrangement direction SD remains substantially unchanged. By reducing the diameter of the locking stent such that the orientation of the struts remains substantially unchanged, the reduction of diameter can be achieved while a state in which the struts are arranged substantially in parallel is maintained. Thus, the diameter of the stent can be reduced to prevent the struts 22a and 22c from intersecting with each other. Due to this effect, the locking stent 2 reduced in diameter and accommodated in the first catheter 5 is less likely to increase in diameter, and therefore, has excellent slidability with respect to the inner wall of the first catheter 5. Due to such excellent slidability, the locking stent 2 is easily pushed toward the distal side D2, and also excels in being resheathed into the first catheter 5 after delivering the second catheter 6 to a target position. In the cells 20 forming the main body 11, the struts 22a are aligned in parallel to the cell arrangement direction SD. This configuration, in which a propulsive force generated by an insertion operation performed by a practitioner is easily transmitted in the cell arrangement direction SD, excels in delivering the locking stent 2 toward the distal side D2.

A closed cell portion 24 is a portion belonging to each cell 20 and having a protruding closed end 25. As illustrated in FIG. 3A, the protruding closed end 25 is an end portion in which a strut 22b and a strut 22d are connected to each other at their proximal side D1 ends, but does not constitute a protruding free end because another strut 22c is connected to the protruding closed end 25. Furthermore, a distal-side D2 end of the strut 22b forms part of a protruding free end 23, and the proximal side D1 end of the strut 22b forms part of the protruding closed end 25. The shape of the protruding closed end 25includes, for example, a substantially V-shape, a substantially U-shape, or a substantially Ω-shape. It may be sufficient for the protruding closed end 25 to protrude substantially toward the proximal side D1. As described above, the protruding closed end 25 of the closed cell portion 24 protrudes toward the proximal side D1, but is inhibited from outwardly warping by the strut 22c. Accordingly, the protruding closed end 25 is less likely to become an obstacle when the locking stent is resheathed (accommodated) into the first catheter 5.

The main body 11 can be produced, for example, by performing laser processing on a tube made of a biocompatible material, preferably a superelastic alloy. In the case of producing the main body 11 from a superelastic alloy tube, it may be preferable that a tube of about 2 mm to 3 mm is subjected in series to: laser processing, expansion to a desired diameter; and shape memory treatment. The production of the main body 11 is not limited to laser processing, but the main body 11 may be produced by cutting processing or the like, or by braiding a wire-shaped metal into a tubular shape.

The main body 11 may be preferably made of a material having high rigidity and high biocompatibility. Examples of such a material include titanium, nickel, stainless steel, platinum, gold, silver, copper, iron, chromium, cobalt, aluminum, molybdenum, manganese, tantalum, tungsten, niobium, magnesium, calcium, and alloys containing these. Furthermore, as such a material, for example, a synthetic resin material of a polyolefin such as polyethylene (PE) or polypropylene (PP), polyamide, polyvinyl chloride, polyphenylene sulfide, polycarbonate, polyether, polymethyl methacrylate, or the like may be used. Moreover, as such a material, for example, a biodegradable resin (biodegradable polymer) such as polylactic acid (PLA), polyhydroxybutyrate (PHB), polyglycolic acid (PGA), or poly ε-caprolactone may be used.

Among the foregoing materials, titanium, nickel, stainless steel, platinum, gold, silver, copper, magnesium, or alloys containing these may be preferable. Examples of the alloys include a Ni-Ti alloy, a Cu-Mn alloy, a Cu-Cd alloy, a Co-Cr alloy, a Cu-Al-Mn alloy, an Au-Cd-Ag alloy, and a Ti-Al-V alloy. A further example of the alloys includes an alloy of magnesium and Zr, Y, Ti, Ta, Nd, Nb, Zn, Ca, Al, Li, Mn, or the like. Among these alloys, a Ni-Ti alloy is preferable.

Referring back to FIG. 1, the delivery wire 3 is a member used when the locking stent 2 is advanced or retracted in the biological lumen. The delivery wire 3 is fed toward the distal side D2 when the locking stent 2 is advanced within a biological lumen V, and is drawn toward the proximal side D1 when the locking stent 2 is retracted within the biological lumen V. The delivery wire 3 is made of, for example, a metal material having a high elastic modulus, such as stainless steel. The delivery wire 3 may have any diameter as long as it has physical properties sufficient to enable advancing and retracting operations to be performed in the biological lumen V and is adapted to the first catheter 5. The diameter of the delivery wire 3 may be, for example, 0.127 mm (0.005 inches) to 0.4572 mm (0.018 inches). A linear delivery member is not limited to a metal material such as a delivery wire, and may be made of, for example, a resin or a composite material of metal and resin.

Among the plurality of catheters including the second catheter 6, a catheter having an inner diameter larger than the first catheter 5 is also referred to as a treatment catheter. The treatment catheter has an inner diameter sufficient to insert a treatment device therein or an inner diameter sufficient to use the treatment catheter itself as a treatment device. The treatment catheter may also be referred to as a guiding catheter in the application in which the treatment device is inserted into the treatment catheter. Examples of the treatment device include a thrombus aspiration device, a flow diverter, an aneurysm embolization device, a thrombectomy device (such as a stent retriever), a stent for treating aneurysm, an aneurysm coil-supporting device, a stent for treating intracranial arterial stenosis, a balloon catheter, a shunt, and a liquid embolic substance release means (such as a catheter having a lumen through which a liquid embolic substance passes). The treatment catheter itself may be used as a treatment device. In such an application, the treatment catheter may also be referred to as a thrombus aspiration catheter. In the embodiment described below, a case where the second catheter 6 is a treatment catheter will be described as an example.

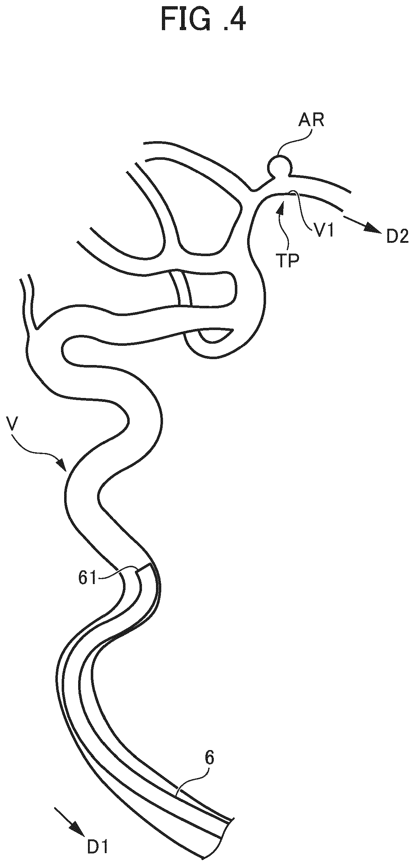

Next, a mode of use of the delivery system 10 including the distal stabilizer 1 of the present embodiment will be described. FIGS. 4 to 11 are schematic diagrams each illustrating one example of a surgical procedure performed using the delivery system 10 including the distal stabilizer 1. Descriptions will be given here of an example in which an aneurysm that has formed at a target position in a biological lumen V is treated. In the present embodiment, the target position is an aneurysm that forms in a distal site in a tortuous blood vessel having a small diameter, such as an intracranial blood vessel, examples of which include an anterior cerebral artery (ACA), an anterior communicating artery (Acom), a middle cerebral artery (MCA), and the like.

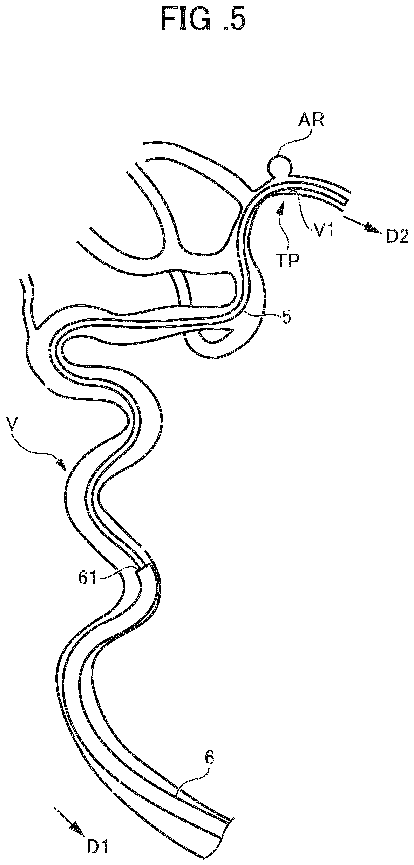

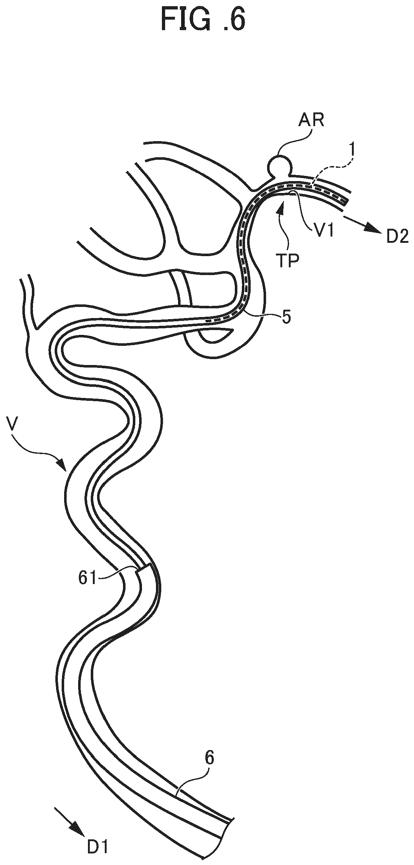

First, the second catheter 6 is placed on the proximal side D1 of the biological lumen V of a patient. As illustrated in FIG. 4, a distal end 61 of the second catheter 6 having a large diameter is likely to be caught by a bent portion or a branching portion of the biological lumen V with a small diameter, and it is difficult to advance the second catheter 6 toward the distal side beyond these portions. As illustrated in FIG. 5, the second catheter 6, into which the first catheter 5 has been inserted, is fed into the biological lumen V. Ater the first catheter 5 is pushed out of the distal end 61 of the second catheter 6, a distal end 51 of the first catheter 5 is placed on a distal side of the target position TP. Next, as illustrated in FIG. 6, the distal stabilizer 1 is inserted into the first catheter 5, and the locking stent 2 is placed on the distal side D2 of the target position TP. During the above-described operation, the locking stent 2 remains accommodated in the first catheter 5 while being in a diameter reduced state.

In the distal stabilizer 1 of the present embodiment, a strut 22a (see FIG. 3A) of one cell 20 is arranged substantially parallel to a strut 22c of another cell 20 adjacent side by side with respect to the cell arrangement direction SD. Due to this configuration, the overlap of struts with each other is reduced when the locking stent 2 is reduced in diameter, and the diameter of the locking stent 2 is less likely to increase. Accordingly, it is possible to suppress deterioration of the slidability with respect to the inner wall of the first catheter 5. As a result, the locking stent 2 is easily pushed toward the distal side D2. Furthermore, in the distal stabilizer 1 of the present embodiment, a strut 22a of each cell 20 is aligned in parallel to the cell arrangement direction SD. This configuration, in which a propulsive force generated by the inserting operation performed by a practitioner is easily transmitted in the cell arrangement direction SD, excels in delivering the locking stent 2 toward the distal side D2. For convenience, FIG. 6 depicts the distal stabilizer 1 (the locking stent 2 + the delivery wire 3) by a broken line only in a distal side portion of the first catheter 5 although the distal stabilizer 1 is accommodated in an entirety of the first catheter 5.

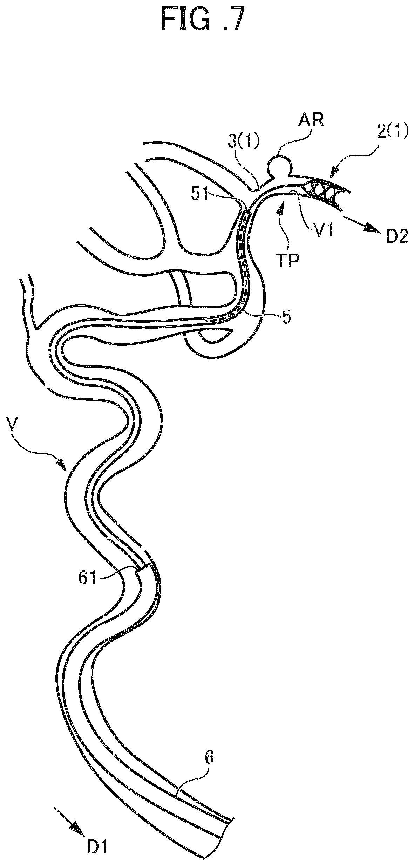

Next, as illustrated in FIG. 7, the locking stent 2 accommodated in the first catheter 5 in the reduced diameter state is released from the distal end 51 of the first catheter 5. Release of the locking stent 2 is effected by an operation of retracting the first catheter 5 toward the proximal side D1. The locking stent 2 released from the distal end 51 of the first catheter 5 expands in diameter due to its self-expanding force. As a result, the locking stent 2 pushes an inner wall (blood vessel wall) V1 of the biological lumen V from inside toward outside to be locked to the inner wall V1. Since the locking stent 2 of the present embodiment includes the open cell portions 21 in each of which the protruding free end 23 (see FIG. 3A and FIG. 3B) protrudes, the locking stent 2 can be locked to the inner wall V1 with a strong locking force. In addition, since the locking stent 2 (main body 11) has a mesh pattern structure in which the plurality of cells 20 are arranged helically with respect to the longitudinal direction LD of the locking stent 2 (see FIG. 2), the locking stent 2 has a high flexibility and facilitates the distal stabilizer 1 to follow bends of the biological lumen V.

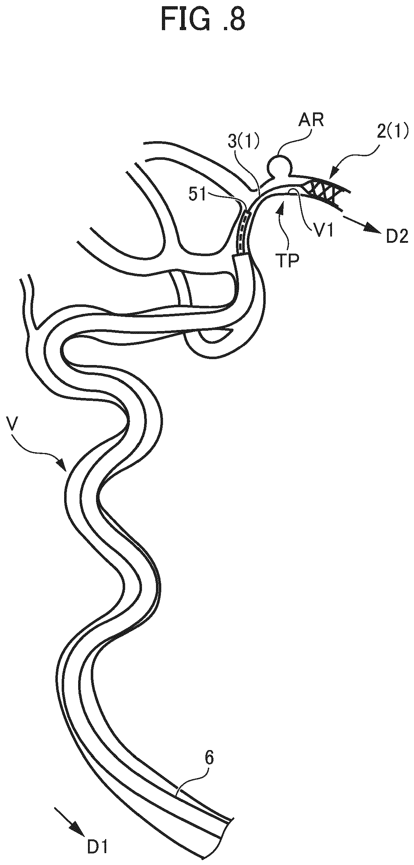

Next, as illustrated in FIG. 8, the second catheter 6 fitted externally over the first catheter 5 is advanced along the first catheter 5 toward the distal side D2. The second catheter 6 has a large outer diameter and high rigidity. For this reason, it becomes more difficult to advance the second catheter 6 as it approaches toward the distal side in the biological lumen having bends. Accordingly, a pulling force is sometimes applied to the inserted first catheter 5 and the delivery wire 3, so that the transmission of a force for advancing the second catheter 6 is improved through straightening the route and the force is produced through removal of deflection. Specifically, in the case where it is difficult to advance the second catheter 6, the locking stent 2 is locked to the inner wall V1 of the biological lumen V, and then, the delivery wire 3 having its distal end fixed is pulled toward the proximal side D1. As a result, the inserted first catheter 5 and the delivery wire 3 are made to pass through the straightened and shortest route. In addition to these, the deflection is removed, whereby the second catheter 6 can be advanced toward the distal side D2.

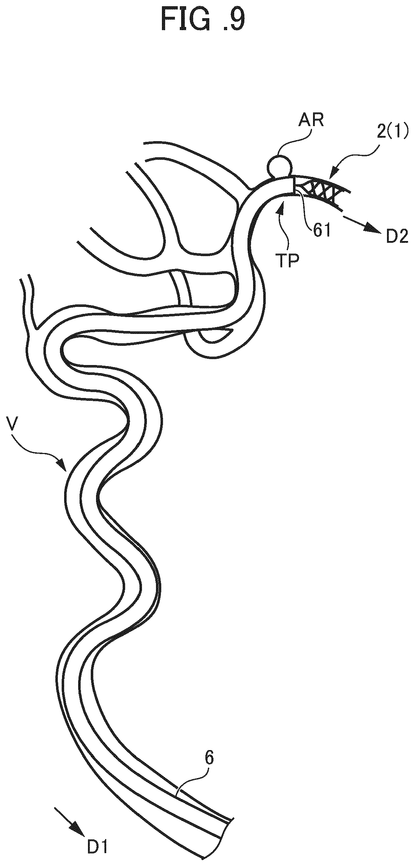

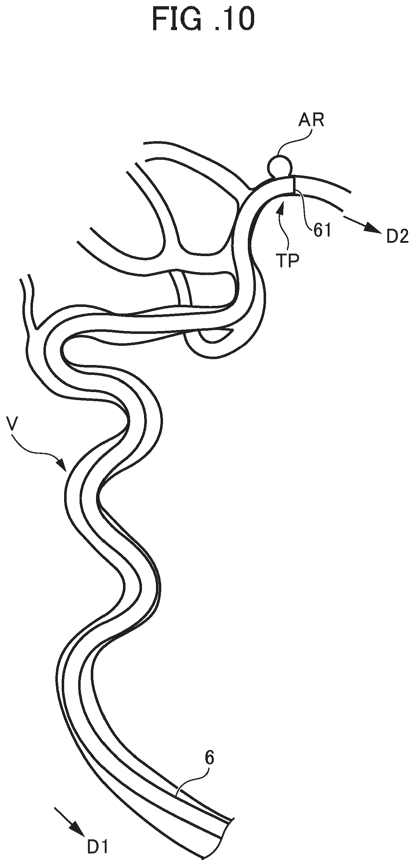

As illustrated in FIG. 9, the distal end 61 of the second catheter 6 can be placed in the vicinity of the aneurysm AR by advancing the distal end 61 of the second catheter 6 within the biological lumen V. After the second catheter 6 is delivered to the vicinity of the target position TP in this way, as illustrated in FIG. 10, the distal stabilizer 1 (locking stent 2) is retracted toward the proximal side D1 to be resheathed into the first catheter 5 (not shown). After the distal stabilizer 1 is resheathed into the first catheter 5, the first catheter 5 and the distal stabilizer 1 may be removed from the second catheter 6 on the proximal side D1. Alternatively, after the first catheter 5 is removed on the proximal side D1, the distal stabilizer 1 may be accommodated in the second catheter 6 and removed on the proximal side D1. In the distal stabilizer 1 of the present embodiment, a strut 22a (see FIG. 3A) of one cell 20 is arranged substantially parallel to a strut 22c of another cell 20 adjacent side by side with respect to the cell arrangement direction SD. Due to this configuration, since the overlap of the struts with each other is reduced when the locking stent 2 is reduced in diameter and the diameter of the locking stent 2 is less likely to increase, the locking stent 2 excels in being resheathed into the first catheter 5 after the second catheter 6 is delivered to the target position.

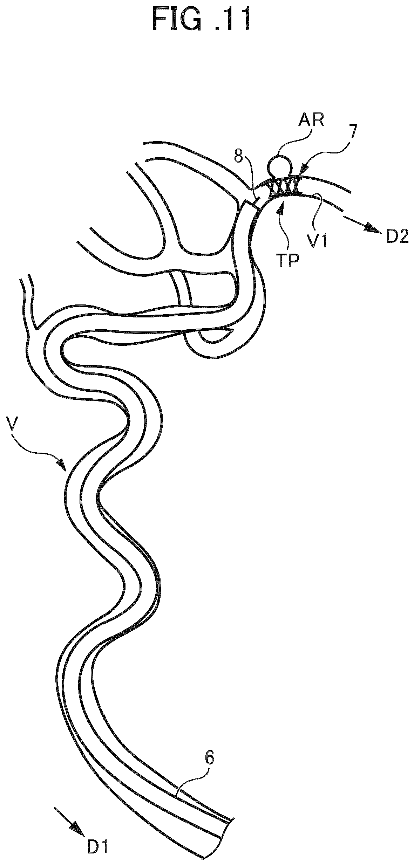

Next, although not shown, an indwelling stent 7 in a reduced diameter state is inserted into the second catheter 6 from the proximal side D1. The indwelling stent 7 is a stent for treating aneurysm. Thereafter, as illustrated in FIG. 11, the indwelling stent 7 is delivered toward the distal side D2 using a delivery wire 8, and the indwelling stent 7 is expanded at the target position TP to indwell there. The purpose of making the indwelling stent 7 indwell at the target position TP is, for example, to reduce blood flowing into the aneurysm AR present at the target position TP or to retain an embolic coil indwelling in the aneurysm AR. After the indwelling stent 7 is made to indwell at the target position TP, an embolic coil (not shown) is delivered toward the distal side D2 through a microcatheter (not shown) inserted in the second catheter 6, whereby the embolic coil can pass through the meshes of the indwelling stent 7 to indwell in the aneurysm AR.

The distal stabilizer 1 of the present embodiment exerts the following effects, for example. In the distal stabilizer 1, a strut 22a of one cell 20 is arranged substantially parallel to a strut 22c of another cell 20 adjacent side by side with respect to the cell arrangement direction SD. Due to this configuration, the overlap of the struts with each other is reduced when the locking stent 2 is reduced in diameter, and the diameter of the locking stent 2 is less likely to increase, thereby making it possible to suppress deterioration of the slidability with respect to the inner wall of the first catheter 5. Therefore, the distal stabilizer 1 of the present embodiment has a frictional force with a blood vessel wall that is necessary for the distal stabilizer 1 as an anchoring device for delivering the second catheter 6 having a large diameter, and also exhibits excellent slidability when inserted into the first catheter 5 with a small diameter while being in a reduced diameter state.

Since the distal stabilizer 1 of the present embodiment has excellent slidability, it can be suitably used for a small-diameter catheter (e.g., a microcatheter having an inner diameter of 0.43 mm (0.017 inches) or less, preferably 0.42 mm (0.0165 inches) or less). As a result, the distal stabilizer 1 of the present embodiment enables delivery of a large-diameter catheter and various treatment devices into a blood vessel having a small diameter such as an anterior cerebral artery (ACA), an anterior communicating artery (Acom), and the like in a cranium, which has been difficult according to the known art. It should be noted that the inner diameter of the catheter to which the distal stabilizer 1 adapted is not particularly limited and may be, for example, greater than 0.43 mm (0.017 inches).

By virtue of the above-described configuration of the present embodiment, the slidability of the distal stabilizer 1 with respect to the inner wall of the first catheter 5 is less likely to deteriorate, and as a result, the distal stabilizer 1 excels in being resheathed into the first catheter 5 after delivering the second catheter 6 to the target position. In the distal stabilizer 1 of the present embodiment, the struts 22a of the cells 20 are aligned parallel to the cell arrangement direction SD. By virtue of this configuration, the propulsive force applied to the delivery wire 3 by a practitioner is easily transmitted in the cell arrangement direction SD, whereby the distal stabilizer 1 excels also in delivering the locking stent 2 toward the distal side D2.

The distal stabilizer 1 of the present embodiment has a mesh pattern structure in which the plurality of cells 20 are arranged helically with respect to the longitudinal direction LD of the locking stent 2. Accordingly, the distal stabilizer 1, which has a high flexibility, follows easily the bends of the biological lumen V. As described above, since the distal stabilizer 1 easily follows the bends of the biological lumen V, stress is less likely to concentrate on opposite ends of the locking stent 2 in the longitudinal direction LD. As a result, an open cell structure that is very gentle on a blood vessel wall is achieved, which is less likely to straighten the blood vessel to which the locking stent 2 is locked.

Although the embodiments described above and the modified embodiments described below can be appropriately combined for use, detailed description thereof will be omitted.

A locking stent 2 may have a mesh pattern structure including cells each having an open cell portion 21 and cells each having only a closed cell portion 24 without an open cell portion 21. In that case, it is desirable to arrange the cells having the open cell portion 21 on the distal side D2 of the locking stent 2.

A locking stent 2 may have a structure in which the cell configuration illustrated in FIG. 3A and the cell configuration illustrated in FIG. 3B are mixed. For example, cells 20 arranged in a region on the proximal side D1 from substantially the middle of the effective length of the locking stent 2 may have the configuration illustrated in FIG. 3A, and cells 20 arranged in a region on the distal side D2 from substantially the middle of the effective length of the locking stent 2 may have the configuration illustrated in FIG. 3B.

In a locking stent 2 illustrated in FIG. 3B, the direction in which a strut 22a intersects with an adjacent strut 22c which lies side by side with the strut 22a may be oriented toward the distal side D2. A main body 11 of a locking stent 2 may have a mesh pattern structure in which a plurality of cells are arranged in parallel with each other along the longitudinal direction LD of the locking stent 2.

### EXPLANATION OF REFERENCE NUMERALS

1: Distal stabilizer
2: Locking stent
3: Delivery wire
5: First catheter
6: Second catheter
10: Delivery system
11: Main body
12: Antenna portion
20: Cell
21: Open cell portion
22 (22a-22e): Strut
23: Protruding free end
24: Closed cell portion
25: Protruding closed end

## Claims

1. A distal stabilizer (1) for use for catheter delivery in a biological lumen (V), the distal stabilizer (1) comprising:
a linear delivery member (3); and
a cylindrical part (2) extending from a distal end of the linear delivery member (3) and configured to be locked to an inner wall of the biological lumen (V), wherein
the cylindrical part (2) has a structure in which cells (20) each having a shape surrounded by wire-shaped members (22) are arranged along a longitudinal direction (LD),
at least one of the cells (20) is an open cell having at least one protruding free end (23), **characterized in that**
a wire-shaped member (22a) forming the protruding free end (23) of the cell (20) and extending in the arrangement direction (SD), when the wire-shaped member (22) is in a natural state, is parallel or intersects at an angle of 0° to 15° with the one of the wire-shaped members (22c) forming another cell (20) that is adjacent side by side to the open cell with respect to the arrangement direction (SD).

2. The distal stabilizer (1) according to claim 1, wherein
the cylindrical part (2) has a structure in which cells (20) each having a shape surrounded by wire-shaped members (22) are arranged helically with respect to the longitudinal direction (LD).

3. The distal stabilizer (1) according to claim 1, wherein
the cylindrical part (2) is used by being inserted into a catheter having an inner diameter of 0.43mm (0.017 inches) or less.

## Patentansprüche

1. Distaler Stabilisator (1) zur Verwendung für die Katheterabgabe in einem biologischen Lumen (V), wobei der distale Stabilisator (1) umfasst:
ein lineares Abgabeelement (3); und
ein zylindrisches Teil (2), das sich von einem distalen Ende des linearen Abgabeelements (3) erstreckt und dazu ausgelegt ist, an eine Innenwand des biologischen Lumens (V) verriegelt zu werden, wobei
das zylindrische Teil (2) eine Struktur aufweist, in der Zellen (20), die jeweils eine Form aufweisen, die von drahtförmigen Elementen (22) umgeben ist, entlang einer Längsrichtung (LD) angeordnet sind,
wenigstens eine der Zellen (20) eine offene Zelle mit wenigstens einem vorstehenden freien Ende (23) ist,
**dadurch gekennzeichnet, dass**
ein drahtförmiges Element (22a), das das vorstehende freie Ende (23) der Zelle (20) bildet und in der Anordnungsrichtung (SD) verläuft, wenn sich das drahtförmige Element (22) in einem natürlichen Zustand befindet, mit dem einen der drahtförmigen Elemente (22c), die eine weitere Zelle (20) bilden, die zu der offenen Zelle bezogen auf die Anordnungsrichtung (SD) Seite an Seite benachbart ist, parallel ist oder es in einem Winkel von 0° bis 15° schneidet.

2. Distaler Stabilisator (1) nach Anspruch 1, wobei:
das zylindrische Teil (2) eine Struktur aufweist, in der Zellen (20), die jeweils eine Form aufweisen, die von drahtförmigen Elementen (22) umgeben ist, bezogen auf die Längsrichtung (LD) helikal angeordnet sind.

3. Distaler Stabilisator (1) nach Anspruch 1, wobei:
das zylindrische Teil (2) verwendet wird, indem es in einen Katheter mit einem Innendurchmesser von 0,43 mm (0,017 Inch) oder weniger eingeführt wird.

## Revendications

1. Stabilisateur distal (1) destiné à être utilisé pour la pose d'un cathéter dans une lumière biologique (V), le stabilisateur distal (1) comprenant :
un élément de pose linéaire (3) ; et
une partie cylindrique (2) s'étendant à partir d'une extrémité distale de l'élément de pose linéaire (3) et configurée pour être verrouillée à une paroi interne de la lumière biologique (V),
la partie cylindrique (2) ayant une structure dans laquelle des cellules (20) ayant chacune une forme entourée par des éléments en forme de fils (22) sont agencées le long d'une direction longitudinale (LD),
au moins une des cellules (20) étant une cellule ouverte ayant au moins une extrémité libre saillante (23),
**caractérisée en ce que**
un élément en forme de fil (22a) formant l'extrémité libre saillante (23) de la cellule (20) et s'étendant dans la direction d'agencement (SD), lorsque l'élément en forme de fil (22) est dans un état naturel, est parallèle ou se coupe selon un angle de 0° à 15° avec l'un des éléments en forme de fils (22c) formant une autre cellule (20) adjacente côte à côte par rapport à la direction d'agencement (SD).

2. Stabilisateur distal (1) selon la revendication 1, la partie cylindrique (2) ayant une structure dans laquelle des cellules (20) ayant chacune une forme entourée par des éléments en forme de fils (22) sont agencées de manière hélicoïdale par rapport à une direction longitudinale (LD).

3. Stabilisateur distal (1) selon la revendication 1, la partie cylindrique (2) est utilisée en étant insérée dans un cathéter d'un diamètre intérieur de 0,43 mm (0,017 pouce) ou moins.
